(19) Europäisches Patentamt
European Patent Office
Office européen des brevets

(11) Veröffentlichungsnummer: **0 247 277 B1**

(12) **EUROPÄISCHE PATENTSCHRIFT**

(45) Veröffentlichungstag der Patentschrift: **11.09.91**

(51) Int. Cl.5: **C07D 213/80**, C07D 213/807, //C07D215/26,C07D215/18

(21) Anmeldenummer: **87101485.8**

(22) Anmeldetag: **04.02.87**

Die Akte enthält technische Angaben, die nach dem Eingang der Anmeldung eingereicht wurden und die nicht in dieser Patentschrift enthalten sind.

# (54) Verfahren zur Herstellung von 5-Alkylchinolinsäuren und Mittel zur Durchführung des Verfahrens.

(30) Priorität: **30.04.86 DE 3614756**

(43) Veröffentlichungstag der Anmeldung:
**02.12.87 Patentblatt 87/49**

(45) Bekanntmachung des Hinweises auf die Patenterteilung:
**11.09.91 Patentblatt 91/37**

(84) Benannte Vertragsstaaten:
**BE CH DE FR GB IT LI NL**

(56) Entgegenhaltungen:
**EP-A- 0 082 542**
**EP-A- 0 104 389**
**EP-A- 0 132 714**
**EP-A- 0 153 908**
**DE-A- 1 545 540**
**DE-B- 1 186 861**
**DE-C- 455 386**
**DE-C- 945 147**
**US-A- 3 351 525**

**CHEMICAL ABSTRACTS, Band 51, Nr. 8, 25. April 1957, Spalten 5768a - 5769c, Columbus, Ohio, US; V. Oakes et al.: "Polyazanaphthalenes. IV. Further derivatives of 1,3,5- and 1,3,8-triazanaphthalene"**

(73) Patentinhaber: **RÜTGERSWERKE AKTIENGESELLSCHAFT**
**Mainzer Landstrasse 217**
**W-6000 Frankfurt am Main 1(DE)**

(72) Erfinder: **Pastorek, Emmerich, Dr.**
**Grünbergerstrasse 90**
**W-6944 Hemsbach(DE)**
Erfinder: **Orth, Winfried, Dr.**
**Am Schachtelgraben 28**
**W-6733 Hassloch/Pfalz(DE)**
Erfinder: **Jeromin, Günter Erich, Dr.**
**Bergstrasse 14**
**W-6900 Heidelberg(DE)**
Erfinder: **Fickert, Werner, Dr.**
**Stockacher Strasse 14**
**W-6800 Mannheim 61(DE)**

**EP 0 247 277 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

HOUBEN-WEYL, Methoden der Organischen Chemie, 4. Auflage, Band IV/1a, 1981, Seiten 478-488, Georg Thieme Verlag, Stuttgart; A. Weickmann et al.: A2) Halogen (V) - Verbindungen als Oxidationsmittel"

CHEMICAL ABSTRACTS, Band 44, Nr. 4, 25. Februar 1950, Abstract Nr. 1505e-h, Columbus, Ohio, US; T. Sorm et al.: "Synthesis of pseudoconhydrine. I. Synthesis of one isomeride of racemic 2-prpyl-5-hydroxypiperidine"

HOUBEN-WEYL, Methoden der Organischen Chemie, 4. Auflage, Band IV/1b, 1975, Seiten 422-424, Georg Thieme Verlag, Stuttgart; A. Stössel "Wismut- und Vanadin-Verbindungen als Oxidationsmittel"

CHEMICAL ABSTRACTS, Band 52, Nr. 3, 10. Februar 1958, Spalte 2007c-1, Columbus, Ohio, US; F.H. Case et al.: "Substituted 1,10-phenanthrolines. X. Ethyl derivatives"

CHEMICAL ABSTRACTS; Band 59, Nr. 12, 9. Dezember 1963, Spalte 13994b-e, Columbus, Ohio, US; G.E. Calf et al.: "Doebner-Miller-Skramp and related reactions III Preparation of some substituted 1,10-phenanthrolines"

CHEMICAL ABSTRACTS, Band 106, Nr. 1, 5. Januar 1987, Abstract Nr. 4836j, Seite 455, Spalte 1, Columbus, Ohio, US; R. Todoriki et al.: "Preparation of 3-substituted quinolines. II. Preparation and cyclodehydration of alpha-alkyl- and alpha-phenyl-béta-arylaminoacrolein derivatives"

## Beschreibung

Die Erfindung betrifft ein neues Verfahren zur Herstellung von 5-Alkyl-2,3-pyridindicarbonsäuren oder 5-Alkylchinolinsäuren sowie neue Verbindungen, aus denen 5-Alkylchinolinsäuren hergestellt werden können. 5-Methylchinolinsäure ist ein wertvolles Ausgangsprodukt für die Herstellung eines Pflanzenschutzmittels. Die Herstellung der 5-Methylchinolinsäure erfolgt bislang durch Umsetzung des 3-Methyl-8-hydroxychinolins mit $HNO_3$. Oakes und Rydon, J.Chem.Soc. 1956, 4433-8 (C.A. (1957) 51:5768d), beschrieben die Oxidation von 8-Hydroxy-3-methylchinolin mit rauchender Salpetersäure zu 5-Methylchinolinsäure.

Nach EP-A 0 153 908 werden 5-Halogen-8-hydroxychinoline zu Pyridin-2,3-dicarbonsäure oxidiert, wobei als Oxidationsmittel Salpetersäure dient unter Verwendung einer Vanadinverbindung als Katalysator.

Ebenfalls mit Salpetersäure in Anwesenheit von Vanadinpentoxid oxidierten Sorm und Sicher, Collection Czechoslov.Chem.Communs 14 (1949) 331-44 (C.A. (1950) 44:1505f) 2-Propyl-8-hydroxychinolin zu 6-Propyl-2,3-pyridindicarbonsäure.

Diese Verfahren versagen bei Chinolinderivaten, die in 3-Stellung mit einer höheren Alkylgruppe substituiert sind. Hier erfolgt keine oxidative Spaltung des Benzolrings, sondern eine Nitrierungsreaktion.

Andererseits war zu erwarten, daß zu den o. g. analoge Pflanzenschutzmittel, die mit 5-Alkylchinolinsäuren mit höherer Alkylgruppe ($C_2$- bis $C_4$-) hergestellt werden, eine stärkere biologische Wirksamkeit haben als solche auf Basis von 5-Methylchinolinsäure. Diese Vermutung wurde durch entsprechende Vorversuche bestätigt.

Es ist daher Aufgabe der vorliegenden Erfindung, ein Verfahren bereitzustellen, mit dem die 5-Alkylchinolinsäuren, insbesondere solche mit einer Alkylkette mit 2 bis 4 C-Atomen, aus leicht zugänglichen Rohstoffen in einem einfachen Prozeß in guter Ausbeute und Reinheit hergestellt werden können.

Die Lösung der Aufgabe erfolgt durch ein Verfahren gemäß der Ansprüche 1 und 2 sowie durch Bereitstellung von dazu notwendigen Verbindungen gemäß Anspruch 3.

Aus EP-A 0 082 542 ist bekannt, im Benzolkern substituierte Chinolinderivate mit Chlorationen in Gegenwart von Vanadyl(V)-Kationen als Katalysator zu Chinolinsäure zu oxidieren.

Es wurde gefunden, daß analog hierzu im Benzolkern substituierte 3-Alkylchinolinderivate mit Chlorationen in Gegenwart von Vanadyl(V)-Kationen als Katalysator zu den entsprechenden 5-Alkylchinolinsäuren oxidiert werden, auch wenn als Alkylgruppe eine höhere und damit reaktivere als die Methylgruppe vorliegt. Dadurch gewinnt eine Verbindungsklasse an Bedeutung, von der bislang ebenfalls nur Methyl-derivate bekannt sind: die im Benzolring substituierten 3-Alkylchinoline mit einer Alkylkette mit 2 bis 4 C-Atomen.

Als für das erfindungsgemäße Verfahren geeignete Alkylchinolinderivate sind alle solche Chinoline, die in 3-Stellung mit einer Alkylgruppe mit 1 bis 4 C-Atomen, d.h. wahlweise mit einer Methyl-, Ethyl-, Propyl-, Isopropyl-, Butyl-, Isobutyl- oder tert. Butylgruppe substituiert sind und in denen am Benzolkern ein oder zwei Wasserstoffatome durch eine aktivierende Gruppe substituiert sind. Aktivierende Gruppen im Sinne der Erfindung sind die Substituenten -OR, -SR, -NRR′, $(-N^{\oplus}(R)_3)^+$, -NH-NRR′, -COOH oder NHCOR, wobei R und R′ gleich oder verschieden sein können und Wasserstoff oder substituierte oder unsubstituierte Alkyl-, Aralkyl- oder Cycloalkylreste mit bis zu 8 C-Atomen bedeuten.

Es ist für die Durchführung des Verfahrens nicht entscheidend, ob und gegebenenfalls mit welchen Gruppen noch weitere H-Atome am Benzolkern substituiert sind. So können sich wahlweise zusätzliche aktivierende Gruppen und/oder nicht aktivierend wirkende Substituenten am Benzolring befinden. 3-Alkylchinolinderivate im Sinne dieser Definition sind z.B.: 5-Ethoxy-, 6-Ethoxy-, 7-Ethoxy-, 8-Ethoxy-, 6-Ethoxy-7-brom, 8-Ethoxy-6-nitro-, 5-Brom-8-methoxy-, 7-Brom-6-methoxy-, 5-Brom-8-propoxy-, 5-(2-Diethylamino-ethoxy)-, 6-(2-Diethylamino-ethoxy)-8-nitro-, 5-(2-Piperidino-ethoxy)-, 5-Methoxy-, 6-Methoxy-, 7-Methoxy-, 8-Methoxy-, 6-Methoxy-5, 7-dimethyl-8-nitro, 5,7-Dichlor-8-methoxy-, 5-Mercapto-, 6-Mercapto-, 7-Mercapto-, 8-Mercapto-, 6-Methylmercapto-, 6-Butylmercapto-, 6-Benzylmercapto-, 8-sec.-Butylmercapto-, 5,8-Dimercapto-, 6-Ethoxy-8-mercapto-, 5,7-Dichlor-8-mercapto-, 8-Methyl-5-mercapto-, 7-Methyl-8-mercapto-, 6-Methyl-8-mercapto-, 6-Methoxy-8-mercapto-, 5-Nitro-8-mercapto-, 5-Hydroxy-, 6-Hydroxy-, 7-Hydroxy-, 8-Hydroxy-, 5-Methyl-8-hydroxy-, 6-Methyl-8-hydroxy-, 5-Ethyl-8-hydroxy-, 7-Brom-8-hydroxy-, 5-Chlor-8-hydroxy-, 5,7-Dichlor-8-hydroxy-, 5-Octyl-8-hydroxy-, 5-Nitro-8-hydroxy-, 7-Nitro-8-hydroxy-, 5-Amino-, 6-Amino-, 7-Amino-, 8-Amino-, 5,8-Diamino-, 6,8-Diamino-, 8-Benzylamino-, 6-Methoxy-8-(4-aminopentyl)-amino-, 5-Nitro-7,8-diamino-, 5,7-Dinitro-8-amino-, 8-Amino-6-chlor-, 6-Methoxy-8-amino-, 8-Acetylamino-6-ethoxy-, 6-Acetylamino-8-brom-, 8-Ethylamino-, 8-Benzylamino-6-methoxy-, 8-Butylamino-, 8-Cyclohexylamino-, 6-Diethylamino-, 8-Diethanolamino-, 8-Piperidino-, 6,8-Bisacetylamino-, 8-(4-Methylamino-piperidino)-, 5-Hydrazino-, 6-Hydrazino-, 7-Hydrazino-, 8-Hydrazino-, 8-Methyl-5-sulfonilamino-, 8-(2-Piperidino-ethoxy)-, 5,7,8-Triamino-3-alkylchinolin, 3-Alkylchinolin-5-carbonsäure, 3-Alkylchinolin-8-carbonsäure, 8-Mercapto-3-alkyl-chinolin-5-sulfonsäure, 8-Hydroxy-3-alkyl-chinolin-5-sulfonsäure, 5-Amino-3-alkyl-chinolin-8-sulfonsäure, 5-Amino-3-alkyl-chinolin-6-carbonsäure.

Es wurde gefunden, daß diese in der beschriebenen Weise substituierten 3-Alkylchinolinderivate direkt zu 5-Alkylchinolinsäure oxidiert werden können, wenn man Chlorationen als Oxidationsmittel unter Verwendung katalytischer Mengen von Vanadyl(V)-Kationen einsetzt.

Es ist dabei von Vorteil, daß bei diesem Oxidationsverfahren sowohl einfach substituierte 3-Alkylchinoline, die gezielt dazu synthetisiert werden können, ebenso oxidiert werden wie kompliziert aufgebaute 3-Alkylchinolinderivate, die unter Umständen bei anderen Synthesen als Abfallprodukte anfallen. Dazu kommt, daß im erfindungsgemäßen Verfahren auch beliebige Gemische von 3-Alkylchinolinderivaten zu 5-Alkylchinolinsäure oxidiert werden, solange jedes der eingesetzten 3-Alkylchinolinderivate am Benzolkern mindestens eine aktivierende Gruppe trägt. Dies bedeutet auch, daß an die Reinheit der 3-Alkylchinolinderivate insofern keine Anforderungen gestellt werden, als auch Isomerengemische, die am Benzolkern in unterschiedlicher Position substituiert sind, im Verfahren eingesetzt werden können.

Vorteilhaft ist weiterhin sowohl die einfache Verfahrensdurchführung in wäßrigem Milieu, bei der keine toxischen Reaktionsprodukte anfallen, als auch die Tatsache, daß Chlorate billige Oxidationsmittel mit einem hohen Oxidationsvermögen sind. Es ist ein weiterer Vorteil des Verfahrens, daß man mit den stöchiometrisch notwendigen Mengen an Chlorat eine gute Ausbeute erzielt und in der Praxis lediglich zum schnelleren Beenden der Oxidationsreaktion einen geringen Überschuß an Oxidationsmittel verwendet.

Als Quelle für die Chlorationen können alle wasserlöslichen Chlorate eingesetzt werden. Vorzugsweise werden jedoch die Chlorate der Erdalkali-und Alkalimetalle und des Ammoniums verwendet. Insbesondere eignet sich das gut handhabbare, kristallwasserfreie, aber gut wasserlösliche Natriumchlorat.

Als Katalysator für diese Oxidationsreaktion dienen Vanadyl(V)-kationen. Sie werden gebildet durch Auflösen von $V_2O_5$ oder wasserlöslicher Vanadate im sauren Reaktionsgemisch. Die Menge an Katalysator, die pro Reaktionsansatz eingesetzt wird, liegt zwischen 0,01 und 0,1 g $V_2O_5$ pro Mol an substituiertem Chinolinderivat, wobei naturgemäß eine höhere Katalysatormenge zu einer größeren Reaktionsgeschwindigkeit führt.

Die Reaktion wird in saurem Medium beim Siedepunkt des Reaktionsgemisches durchgeführt, wobei ein entsprechend substituiertes 3-Alkylchinolinderivat oder ein Gemisch mehrerer substituierter 3-Alkylchinolinderivate, Säure und Katalysator in wäßriger Lösung vorgelegt und erwärmt werden. Danach wird unter Rühren das Oxidationsmittel portionsweise zugegeben. Dabei kann festes Chlorat zudosiert werden, daß sich im Reaktionsgemisch löst. Bevorzugt aber wird das Chlorat vorher in Wasser gelöst. Gegen Ende der Oxidationsreaktion bilden sich geringe Mengen gelbgrüner Gase, die gemeinsam mit dem bei der Reaktion entstehenden Kohlendioxid entweichen. Es ist daher zweckmäßig, das entweichende Gas durch eine Wäsche mit z.B. einer alkalischen Natriumsulfitlösung zu reinigen. Nach beendeter Reaktion wird das erhaltene Reaktionsgemisch leicht abgekühlt und der pH-Wert mit Hilfe einer Lauge auf pH 4 - 5,5 eingestellt. In diesem Bereich liegen die 5-Alkylchinolinsäuren als Betaine gelöst vor, während durch Nebenreaktion entstandene Verunreinigungen ungelöst bleiben und, gegebenenfalls nach Adsorption an Aktivkohle, abgetrennt werden können.

Nach dem Ansäuern auf pH 0 - 1 fallen die 5-Alkylchinolinsäuren aus der Reaktionslösung nur schwer und nur teilweise aus.

Es wurde aber gefunden, daß sie als schwerlösliche Kupfersalze quantitativ zu fällen sind. Die Abtrennung von Kupfer erfolgt im alkalischen Medium, in dem das Kupfer als Oxid gefällt und abfiltriert wird. Wird die so erhaltene Mutterlauge mit einer starken Mineralsäure auf pH 0 - 1 angesäuert, so fallen die reinen 5-Alkylchinolinsäuren überraschenderweise leicht und vollständig aus.

## Beispiele

### Beispiel 1
Herstellung von 3-Ethyl-8-methoxychinolin

1040 g (10,5 mol) konzentrierte Salzsäure, 156 g (2,6 mol) Eisessig, 320 g (2,6 mol) o-Anisidin und 199 g (1,3 mol) o-Nitroanisol werden in einem 4 l-Dreihalskolben vorgelegt, zum Rückfluß erhitzt und innerhalb von 2 h werden 328 g (3,9 mol) 2-Ethylacrolein hinzugetropft. Anschließend kocht man noch 2 h unter Rückfluß nach. Nach dem Abkühlen alkalisiert man mit Natronlauge bis zum pH 10 und extrahiert die wäßrige Phase mit 1000 ml Chloroform. Die Chloroformphase wird nach dem Trocknen über $MgSO_4$ eingedampft und der Rückstand unter Vakuum destilliert.
$Kp_6$: 160 - 166 °C; 571 g (78,2 %) gelbes Öl.

### Beispiel 2
Herstellung von 3-Ethyl-8-hydroxychinolin-hydrochlorid

In einem 4 l-Dreihalskolben mit Rührer, Tropftrichter und Rückflußkühler werden 680 ml konzentrierte Salzsäure, 114 ml Eisessig, 220 g (2,02 mol) o-Aminophenol und 145 g (1,04 mol) o-Nitrophenol vorgelegt und das Ganze auf Rückfluß erhitzt. Jetzt werden innerhalb von 2 h 252,4 g (3 mol) 2-Ethylacrolein hinzugetropft. Anschließend rührt man noch 2 h unter Rückfluß nach. Nach dem Abkühlen versetzt man das Reaktionsgemisch mit 800 ml Essigester und rührt 1 - 2 h nach, wobei das Hydrochlorid auskristallisiert. Das Salz wird abgesaugt, mit 500 ml Essigester nachgewaschen und getrocknet.
Ausbeute: 321 g (50 %), Fp. 256 - 258 °C, gelbgrünes Kristallpulver.

Beispiel 3
Herstellung von 3-Ethyl-8-chlorchinolin

400 g (4 mol) konzentrierte Salzsäure, 60 g (1 mol) Eisessig, 127,6 g (1 mol) 2-Chloranilin und 78,8 g (0,5 mol) 1-Chlor-2-nitrobenzol werden in einem 4 l-Dreihalskolben mit Tropftrichter, Rührer und Rückflußkühler vorgelegt und bis zum Rückfluß erhitzt. Jetzt tropft man innerhalb von 2 h 126 g (1,5 mol) 2-Ethylacrolein hinzu. Man rührt noch 2 h unter Rückfluß nach und läßt abkühlen. Anschließend alkalisiert man die Mischung unter Kühlung mit NaOH, 50%ig, bis pH 9 und extrahiert die wäßrige Phase mit 500 ml Essigester. Die Essigesterphase wird abgetrennt, über $Na_2SO_4$ getrocknet und das Lösemittel abdestilliert. Der Rückstand wird im Vakuum destilliert. Nach einem geringen Vorlauf destillierten bei $Kp_{0,4}$ 130 - 134 °C 186 g (64,7 %, bezogen auf eingesetztes 2-Ethylacrolein) 3-Ethyl-8-chlorchinolin.

Beispiel 4
Herstellung von 8-Methoxy-3-methylchinolin

1040 g (10,5 mol) konzentrierte Salzsäure, 156 g (2,6 mol) Eisessig, 320 g (2,6 mol) o-Anisidin werden in einem 4 l-Dreihalskolben vorgelegt, auf Rückfluß erhitzt und innerhalb von 2,5 h eine Lösung von 199 g (1,3 mol) o-Nitroanisol in 328 g (4 mol) 2-Methacrolein, 85%ig, hinzugetropft. Anschließend kocht man noch 3 h unter Rückfluß und läßt abkühlen. Die Mischung wird mit NaOH, 50%ig, leicht alkalisiert und anschließend mit 1000 ml Chloroform extrahiert. Nach dem Abtrennen wir die Chloroformphase eingedampft und der Rückstand im Vakuum destilliert.
$Kp_{0,5}$: 130 - 140 °C; 460 g (66,4 %) gelbes Öl, das rasch erstarrt.
Fp.: 70 - 72 °C (Cyclohexan).

Beispiel 5
5-Ethylchinolinsäure aus 3-Ethyl-8-methoxychinolin

187 g (1 mol) 3-Ethyl-8-methoxychinolin werden in 300 ml Wasser und 100 ml (1,28 mol) Salzsäure, konzentriert, gelöst und 0,2 g Ammoniumvanadat zugegeben. Diese Lösung wird auf 90 - 95 °C erhitzt und innerhalb von 2 - 3 h wird unter Rühren bei 95 -100 °C eine Lösung von 404 g (3,8 mol) Natriumchlorat in 440 ml Wasser zugegeben. Die Reaktion ist mäßig exotherm, und darum muß während der Oxidation noch Wärme zugeführt werden. Nach ca. 15 min Nachreaktion bei 95 - 100 °C folgt eine exotherme Phase, welche ca. 50 min andauert, und während dieser Zeit hält sich die Reaktionstemperatur ohne Wärmezufuhr bei ca. 100 °C.

Nach dieser Nachreaktion werden der Mischung zum Abbau des noch vorhandenen Chlorats 30 g $NaHSO_3$ (oder entsprechende Lösung) hinzugefügt. Anschließend wird die Reaktionsmischung mit 50%iger Natronlauge bis pH 4,5 neutralisiert. Bei pH 3,5 tritt Chloroform auf, welches abdestilliert werden kann. Nun werden 20 g Aktivkohle zugesetzt, die Mischung 15 min gerührt und dann der Feststoff abgesaugt und mit Wasser nachgewaschen. Das resultierende Filtrat wird mit konzentrierter Salzsäure bis pH 1 angesäuert und bei 90 - 95 °C mit 125 g Kupfersulfat versetzt. Nach dem Abkühlen auf 20 - 25 °C wird das Kupfersalz abgenutscht und mit 2 x 100 ml Wasser nachgewaschen. Es werden ~ 150 g Cu-Ethylchinolat erhalten.

Spaltung des Kupfersalzes der 5-Ethylchinolinsäure

Das erhaltene Kupfersalz wird in 250 ml Wasser aufgeschlämmt und unter Rühren mit 182 g 50%iger Natronlauge versetzt. Das Gemisch wird 15 min unter Rückfluß erhitzt. Nach Zugabe von 20 g Celite® wird bei 50 °C abfiltriert. Der Filterkuchen von Cu-Oxid wird mit 2 x 25 ml warmen Wasser nachgewaschen. Das Filtrat wird bei 50 °C mit 70%iger Salpetersäure auf pH 1 gebracht. Die 5-Ethylchinolinsäure wird bei ~ 15 °C abgenutscht, mit 3 x 30 ml Wasser (kalt) nachgewaschen und getrocknet.
Ausbeute: 99 g.

Fp.: 148 - 151 °C.

Beispiel 6
5-Ethylchinolinsäure aus 3-Ethyl-8-hydroxychinoliniumchlorid

210 g (1 mol) 3-Ethyl-8-hydroxychinoliniumchlorid werden in 350 ml Wasser gelöst, 20 ml (0,24 mol) Salzsäure, konzentriert, sowie 0,2 g Ammoniumvanadat zugegeben. Zu dieser, auf ca. 90 °C erhitzten Lösung werden im Laufe von 2 bis 3 h unter Rühren eine Lösung von 335 g (3,15 mol) Natriumchlorat in 365 ml Wasser zugegeben. Die Reaktion ist exotherm und eine Wärmezufuhr ist während der Reaktion nicht erforderlich; die Oxidation verläuft bei ca. 100 °C. Nachher rührt man noch ca. 1 h ohne Heizen nach und stellt den pH-Wert der Reaktionslösung durch vorsichtiges Zutropfen von ~ 115 g (1,44 mol) Natronlauge, 50%ig, auf 4,5 ein. Die ungelösten Nebenprodukte werden abgetrennt, indem 15 g Aktivkohle zugegeben und nach kräftigem Rühren abgenutscht werden. Das erhaltene Filtrat wird mit ~ 90 ml (1,07 mol) Salzsäure auf pH 1 gebracht, die 5-Ethylchinolinsäure analog zu Beispiel 5 als Kupfersalz gefällt, isoliert und aus diesem freigesetzt.
Ausbeute: 90 g 5-Ethylchinolinsäure.

Beispiel 7
5-Ethylchinolinsäure aus 8-Chlor-3-ethylchinolin

192 g 8-Chlor-3-ethylchinolin werden in 400 ml Wasser und 120 g Schwefelsäure gelöst, 0,2 g Ammoniumvanadat zugegeben, auf 95 °C erhitzt und innerhalb von 3 - 4 h wird unter Rühren eine Lösung von 320 g $NaClO_3$ in 350 ml Wasser bei 95 - 100 °C zugegeben. Während der Chloratzugabe muß geheizt werden, um die Reaktionstemperatur aufrechtzuerhalten. Die Nachreaktion wird eine 1/2 h bei 95 °C durchgeführt. Mit ca. 180 g Natronlauge, 50%ig, wird das Reaktionsgemisch auf pH 4,5 eingestellt, mit Aktivkohle gereinigt, angesäuert und die Ethylchinolinsäure analog Beispiel 5 über das Kupfersalz isoliert.
Ausbeute: 74 g 5-Ethylchinolinsäure.

Beispiel 8
5-Methylchinolinsäure aus 8-Methoxy-3-methylchinolin

173 g 8-Methoxy-3-methylchinolin werden in 400 ml Wasser und 120 g Schwefelsäure gelöst, 0,2 g Ammoniumvanadat zugegeben und auf 95 °C erhitzt. Innerhalb von 2 h wird bei 95 - 100 °C unter Rühren eine Lösung von 350 g Natriumchlorat in 400 ml Wasser zugegeben. Während der Oxidation muß geheizt werden, um die Reaktionstemperatur aufrechtzuerhalten. Eine exotherme Reaktion setzt erst nach der Zugabe des Oxidationsmittels ein. Nach Abklingen der exothermen Reaktion wird noch 90 min bei ca. 100 °C nachgerührt. Durch Zugabe von ca. 100 g Natronlauge, 50%ig, wird das Reaktionsgemisch auf pH 4,5 gebracht, über Aktivkohle gereinigt und mit ca. 90 ml Salzsäure auf pH 1 gebracht. Die ausgefallene 5-Methylchinolinsäure wird abgenutscht, gewaschen und getrocknet.
Ausbeute: 146 g 3-Methylchinolinsäure (= 80,6 % d.Th.).

Beispiel 9
5-Methylchinolinsäure aus 8-Hydroxy-3-methylchinolin

159 g 8-Hydroxy-3-methylchinolin werden in 400 ml Wasser und 102 ml Salzsäure, konz., gelöst, 0,2 g Ammoniumvanadat zugegeben und auf 95 °C erhitzt. Innerhalb von 2 h wird bei 95 - 100 °C unter Rühren eine Lösung von 320 g Natriumchlorat in 350 ml Wasser zugegeben. Die Reaktion ist exotherm und eine Wärmezufuhr ist während der Reaktion nicht erforderlich. Danach rührt man noch 2 h bei 100 °C nach. Nach Abkühlen des Reaktionsgemisches auf ca. 80 °C wird mit Natronlauge, 50%ig, auf pH 5,5 eingestellt, mit Aktivkohle gereinigt und mit Salpetersäure auf pH 1 gebracht.
Ausbeute: 148 g 3-Methylchinolinsäure (= 81,7 % d.Th.).

Vergleichsbeispiel 1

3-Ethyl-8-methoxychinolin wird nach der Arbeitsweise von Oakes und Rydon (Soc. 1956, 4433, 4438) mit rauchender Salpetersäure umgesetzt. Es entsteht keine Ethylchinolinsäure. Erhalten wird ein gelbes Produkt. Fp. nach Umkristallisation aus Cyclohexan: 82 - 84 °C. Absorptionsbanden im IR-Spektrum bei 1350 $cm^{-1}$ und 1560 $cm^{-1}$ deuten auf ein Nitrierungsprodukt. Die CHN-Analyse, kalkuliert auf 3-Ethyl-8-methoxynitrochinolin, ergibt folgende Werte:

| | berechnet | gefunden |
|---|---|---|
| C | 62,06 | 61,1 |
| H | 5,21 | 5,1 |
| N | 12,06 | 12,0 |

Vergleichsbeispiel 2

3-Ethyl-8-methoxychinolin wird nach dem aus EP-A 153 908 bekannten Oxidationsverfahren mit Salpetersäure (64%ig) in Gegenwart von Ammoniumvanadat umgesetzt. Es entsteht keine Ethylchinolinsäure. Das Reaktionsprodukt ist ein gelbes Produkt mit einem Fp nach Umkristallisation aus Cyclohexan von 88 - 90 °C. Absorptionsbanden im IR-Spektrum bei 1350 $cm^{-1}$ und 1560 $cm^{-1}$ deuten auf ein Nitrierungsprodukt.

Die CHN-Analyse, kalkuliert auf 3-Ethyl-8-methoxynitrochinolin ergibt folgende Werte:

| | berechnet | gefunden |
|---|---|---|
| C | 62,06 | 61,1 |
| H | 5,21 | 5,1 |
| N | 12,04 | 11,9 |

**Patentansprüche**

1. Verfahren zur Herstellung von 5-Alkylchinolinsäuren, dadurch gekennzeichnet, daß 3-Alkylchinolinderivate der allgemeinen Formel

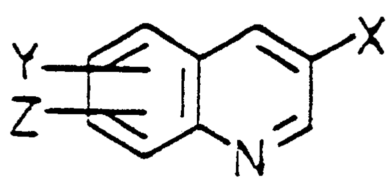

in der X einen verzweigten oder unverzweigten Alkylrest mit 1 bis 4 C-Atomen, Y Wasserstoff oder einen zusätzlichen aktivierenden und/oder nicht aktivierenden Substituenten und Z -OR, -SR, -NRR', Halogen, $(-N^{\oplus}(R)_3)^+$, -NH-NRR', -COOH oder -NHCOR und R und R' gleich oder verschieden sein können und Wasserstoff oder substituierte oder unsubstituierte Alkyl-, Aralkyl- oder Cycloalkylreste mit bis zu 8 C-Atomen bedeuten, in saurem, wäßrigem Medium beim Siedepunkt des Reaktionsgemisches mit Chlorationen in Gegenwart von Vanadyl(V)-Kationen als Katalysator oxidiert werden, wobei die Menge an Vanadyl(V)-Kationen 0,01 bis 0,1 g $V_2O_5$ pro Mol an substituiertem Chinolinderivat entspricht.

2. Verfahren nach Anspruch 1, dadurch gekennzeichnet, daß die 5-Alkylchinolinsäuren im stark sauren Medium mit Kupferionen aus dem Reaktionsgemisch gefällt und als Kupfersalze abgetrennt werden, wonach das Kupfer im alkalischen Bereich als Hydroxid gefällt und abgetrennt und die 5-Alkylchinolinsäuren nach erneutem Ansäuern auf pH 0 - 1 auskristallisiert und rein isoliert werden.

3. 3-Alkylchinolinderivate der allgemeinen Formel

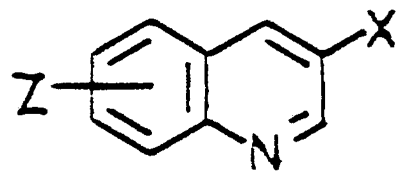

in der X einen verzweigten oder unverzweigten Alkylrest mit 2 bis 4 C-Atomen und Z -OR, SR, -COOH, und R Wasserstoff oder eine Methylgruppe bedeutet und die restlichen Wasserstoffatome am Benzolkern nicht substituiert sind.

## Claims

1. A process for the preparation of 5-alkylquinolinic acids, characterized in that 3-alkylquinoline derivatives of the general formula

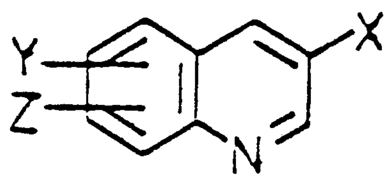

in which X represents a branched or unbranched alkyl residue with from 1 to 4 carbon atoms, Y represents hydrogen or an additional activating and/or non-activating substituent and Z represents -OR, -SR, -NRR', halogen, $(-N^{\oplus}(R)_3)^+$, -NH-NRR', -COOH or NHCOR and R and R' can be the same or different and hydrogen or substituted or unsubstituted alkyl, aralkyl or cycloalkyl residues with up to 8 carbon atoms, are oxidized in an acidic aqueous medium at the boiling point of the reaction mixture with chlorate ions in the presence of vanadyl(V) cations as a catalyst, the quantity of vanadyl(V) cations corresponding to from 0·01 to 0·1 g $V_2O_5$ *per* mol of substituted quinoline derivative.

2. A process according to Claim 1, characterized in that the 5-alkylquinolinic acids are precipitated from the reaction mixture in a strongly acid medium with copper ions and are separated as copper salts, after which the copper is precipitated as hydroxide in the alkaline range and separated and the 5-alkylquinolinic acids are crystallized out after renewed acidification to pH 0 - 1 and are isolated pure.

3. 3-alkylquinoline derivatives of the general formula

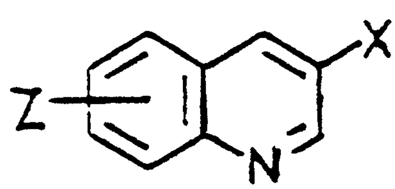

in which X represents a branched or unbranched alkyl residue with from 2 to 4 carbon atoms and Z represents -OR, SR, -COOH, and R represents hydrogen or a methyl group, and the residual hydrogen atoms on the benzene core are not substituted.

## Revendications

1. Procédé pour la préparation d'acides 5-alkylquinoléiques, caractérisé en ce que des dérivés de 3-alkylquinoléine de formule générale

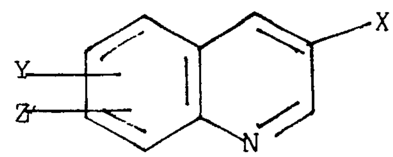

dans laquelle X représente un reste alkyle avec 1 à 4 atomes de C, ramifié ou non ramifié, Y de l'hydrogène ou un substituant activateur et/ou nonactivateur additionnel et Z représente -OR, -SR, -NRR', un halogène, $(-N^{\oplus}(R)_3)^+$, -NH-NRR', -COOH ou -NHCOR et R et R' peuvent être identiques ou différents et signifient de l'hydrogène ou des restes alkyles, aralkyles ou cycloalkyles substitués ou non substitués possédant jusqu'à 8 atomes de C, sont oxydés en milieu acide aqueux au point d'ébullition du mélange réactionnel avec des ions chlorate en présence de cations vanadyle (V) en tant que catalyseur, la quantité de cations vanadyle (V) correspondant à 0,01 à 0,1 g de $V_2O_5$ par mole de dérivé de quinoléine substitué.

**2.** Procédé selon la revendication 1, caractérisé en ce que les acides 5-alkylquinoléiques sont précipités hors du mélange réactionnel en milieu fortement acide avec des ions cuivre et sont séparés sous forme de sels de cuivre, après quoi le cuivre est précipité dans le domaine alcalin sous forme d'hydroxyde et est séparé et les acides 5-alkylquinoléiques sont séparés à l'état cristallin après une acidification renouvelée à pH 0,1 et isolés à l'état pur.

**3.** Dérivés de 3-alkylquinoléine de formule générale

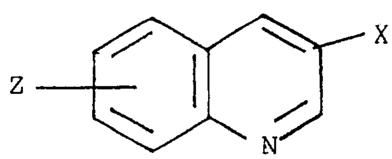

dans laquelle X signifie un reste alkyle ramifié ou non ramifié possédant 2 à 4 atomes de C, Z signifie -OR, -SR, -COOH et R de l'hydrogène ou un groupe méthyle et dans laquelle les atomes d'hydrogène restants sur le noyau benzénique ne sont pas substitués.